# EUROPEAN PATENT SPECIFICATION

(11) **EP 2 152 105 B1**
(45) Date of publication and mention of the grant of the patent: **06.03.2013**
(21) Application number: 08760225.6
(22) Date of filing: 29.05.2008
(51) Int. Cl.: A24F 25/02, A24F 15/18

(54) **HEATABLE HUMIDIFYING DEVICE FOR USE WITH TOBACCO PRODUCTS**
ERHITZBARES ANFEUCHTUNGSGERÄT FÜR DIE VERWENDUNG MIT TABAKPRODUKTEN
DISPOSITIF HUMIDIFICATEUR CHAUFFABLE DESTINÉ À ÊTRE UTILISÉ AVEC LES PRODUITS DE TABAC

(30) Priority: 05.06.2007 GB 0710776
(43) Date of publication of application: 17.02.2010
(73) Proprietor: British American Tobacco (Investments) Limited, London WC2R 3LA (GB)
(72) Inventor: STEPHENSON, Darryl, London WC2R 3LA (GB); SUTTON, Joseph Peter, London WC2R 3LA (GB)
(74) Representative: Gill, Siân Victoria
(86) International application number: PCT/EP2008/056634
(87) International publication number: WO 2008/148699

(56) References cited:
- WO-A-98/41116
- US-A- 2 220 777
- US-A- 4 997 082
- US-A- 5 957 380

## Description

### BACKGROUND OF THE INVENTION

The present invention relates to a heatable humidifying device for use with tobacco products. A prior art device according to the preamble of claim 1 is known from WO-98/4116.6.

The tobacco in products such as cigarettes, cigars and loose tobacco for pipe smoking or roll-your-own cigarettes is prone to drying out both during the pre-use storage period and particularly after the product packaging has been opened. This is undesirable, since tobacco becomes less palatable as it dries.

Expensive cigars are generally stored in specially designed humidors specifically to address this problem by keeping the cigars at a desired level of humidity to prevent drying. A more rudimentary approach is often used by consumers of loose tobacco; a piece of apple or potato peel placed in the tobacco pouch or tin can help to keep the tobacco moist or can re-moisten tobacco that has become too dry. However, this method may not be considered hygienic, and may taint the tobacco with unwanted scents or flavours.

Various humidification devices and moisture dispensers have therefore been proposed for use with tobacco products, in particular for use in tobacco pouches and tins and cigarette packs. Typically, the devices comprise a source of moisture that can emit water vapour which is surrounded by a permeable enclosure such as a housing, casing, layer or pouch which keeps the moisture source separate from the tobacco but allows the water vapour to be transmitted to the tobacco. The moisture source is often some form of water-absorbent medium such as cotton wool, blotting paper, sponge, florists' foam, or polyacrylamide gel or crystals, which is soaked with water or salt solution. The permeable enclosure may be rigid or flexible, and may be made permeable by the provision of perforations or apertures therein, or may be an inherently permeable membrane material.

Examples of such devices can be found in GB 2,345,431, GB 2,265,295, GB 1,369,992, GB 741,475, GB 567,141, GB 308,127, GB 119,489, EP 531,075, EP 363,194, EP 348,840, US 5,957,380, US 5,957,277, US 5,829,452, US 3,801,011, US 1,874,989, US 1,871,419, WO 02/12089, DE 4000143, DE 20 2005 008 520, DE 20 2005 010 286 and FR 2,619,289. Some devices include an outer non-permeable enclosure to prevent moisture loss before the device is used. For example, US 5,957,380 describes a plastic tube containing a porous material holding a humidification solution of glycol, water and saturated salt, the tube having end caps which are removed to let water vapour out when the device is placed in a cigar container. US 3,801,011 describes a humidity control device comprising a sheet-like carrier such as an adhesive tape which carries a layer of water-containing capsules having vapour-transmissive walls, and which is supplied in a sealed plastic bag.

In all of these examples, the release of water vapour is dependent on room-temperature evaporation from the moisture source.

### SUMMARY OF THE INVENTION

A first aspect of the present invention is directed to a humidifier for use with tobacco products, comprising: a first container permeable to the passage of moisturising vapour and configured to enclose a moisture-releasing element that emits moisturising vapour; and a second container arranged for thermal energy transfer to a moisture-releasing element in the first container, the second container having sealed therein: a supercoolable salt hydrate solution; and an activating device actuable to initiate crystallisation of the salt hydrate solution, thereby releasing heat and supplying it to a moisture-releasing element enclosed in the first container.

For example, the first container may be permeable to the passage of water vapour. The moisture-releasing element may then emit water vapour. Alternatively, liquids other than water that evaporate to give moisturising vapour suitable for moisturising tobacco can be used.

Crystallisation of the supercooled salt hydrate solution produces heat energy via the latent heat of crystallisation, which is transferred to the moisture-releasing element where it increase the rate of evaporation and water vapour release from the first container. Water vapour can hence be supplied more rapidly to a tobacco product if a user desires to rehydrate dry tobacco quickly. Otherwise, the humidifier acts in the usual manner by giving off water vapour at a slower rate determined by the evaporation rate at room temperature. Therefore, the user is provided with control over the rate of water vapour supply.

Heating by salt hydrate solution crystallisation is safe, fast and requires no power source, so is well-suited for use with a humidifier for tobacco products which is likely to be small, compact and portable.

The humidifier may be supplied without water (or other liquid), for filling by the user when required, but otherwise, the humidifier further comprises a moisture-releasing element enclosed in the first container. The moisture-releasing element may emit water vapour, so that the moisture-releasing element may comprise a reservoir of liquid water, or alternatively water absorbed in a water-absorbing medium. The water-absorbing medium may be, for example, one or more of polyacrylamide granules, an alginate, microcrystalline cellulose, ispaghula husk, saturated silica gel, rice, a starch gel, a pectin, natural sponge, synthetic sponge, a sepeolite, bentonite or other clay material, gelatin, agar agar, and a modified cellulosic gum.

The second container may be flexible, and/or the first container may be flexible. Flexibility facilitates operation of the activating device by manipulation through the walls of the containers.

In some embodiments, the first container and the second container may be adjacent to one another. For example, the first container and the second container may be separate and coupled together by a bonding material. This may ease manufacture of the containers, which will typically comprise different materials. Further, the bonding material may be reversibly releasable to allow the first container and the second container to be coupled and uncoupled. This allows actions upon the containers, including the possibilities of adding water to the first container and melting and supercooling the salt hydrate solution in the second container, to be conveniently carried out using the individual containers only.

In other embodiments, the first container and the second container share a common wall. This may provide better transfer of heat energy from the salt hydrate solution to the moisture-releasing element, since the heat has only one wall to pass through.

Further in this regard, alternatively, the second container may be inside the first container. Again, there is only one wall for the heat energy to propagate through. Additionally, the heat energy is transferred to the moisture-releasing element over the entire surface of the second containers. None is wasted, and heating of the moisture-releasing element is more efficient.

The humidifier may be configured for more than one release of heat from the salt hydrate solution. Once crystallised, the solution, sealed inside the second container, can be heated above its melting point and then allowed to cool back to room temperature. This happens by supercooling, produced by the confinement of the solution inside the second container, so that the solution remains liquid at room temperature. Crystallisation can then be initiated again for further heat supply.

Heating of the salt hydrate solution can conveniently be carried out by immersing the second container in hot or boiling water. If the humidifier is configured such that the first and second containers are permanently joined, preferably the first container and any moisture-releasing element enclosed in the first container are able to withstand immersion in hot water for the purpose of melting the salt hydrate solution after crystallisation.

According to some embodiments, the moisture-releasing element can be rehydrated. This allows the humidifier to be used multiple times, and takes advantage of the fact that the salt hydrate solution can undergo multiple crystallisations for heat release. For example, the first container may be opened and closed for the purpose of introducing water (or other liquid) into the first container.

The humidifier may further comprise a removable outer layer that is substantially impermeable to the passage of water or water vapour, the outer layer disposed over at least the first container and intended to be removed before use of the humidifier. The outer layer stops loss of water vapour by evaporation and retains the water inside the humidifier until it is required for use.

The removable outer layer may comprise an outer wrapper enclosing both the first container and the second container. Alternatively, the removable outer layer may comprise a peelable layer adhered over at least part of the outer surface of the first container.

A second aspect of the present invention is directed to a package for tobacco products containing one or more tobacco products and a humidifier according to the first aspect.

### BRIEF DESCRIPTION OF THE DRAWINGS

For a better understanding of the invention and to show how the same may be carried into effect reference is now made by way of example to the accompanying drawings in which:
Figure 1 shows a cross-sectional view of a humidifier according to an embodiment of the present invention in which first and second containers of the humidifier share a common wall;
Figure 2 shows a cross-sectional view of a humidifier according to an embodiment in which the first container can be opened for the addition of water;
Figure 3 shows a cross-sectional view of a humidifier according to an embodiment in which the first and second containers are separate and coupled together;
Figure 4 shows a cross-sectional view of a humidifier according to an embodiment in which the second container is positioned inside the first container;
Figure 5 shows a cross-sectional view of a humidifier according to an embodiment which includes a removable impermeable outer wrapper around the humidifier; and
Figure 6 shows a cross-sectional view of a humidifier according to an embodiment which includes a removable impermeable outer layer adhered over the first container.

### DETAILED DESCRIPTION

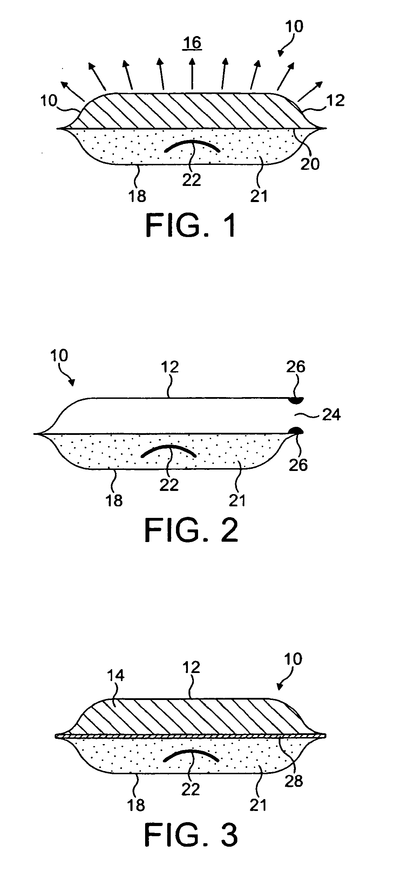

Figure 1 shows a cross-sectional view through a humidifier for use with tobacco products according to an embodiment of the present invention. The humidifier 10 comprises a first container 12 defined by a wall or membrane at least part of which is permeable to the passage of moisturising vapour, but substantially impermeable to the passage of liquid. The first container 12 forms a reservoir for holding a moisture-releasing element 14 that emits moisturising vapour such as water vapour. The porosity properties of the first container mean that it retains the moisture releasing element 14 and prevents the loss of liquid therefrom, but allows moisturising vapour 16 given up by the moisture-releasing element via evaporation to pass through to the surrounding environment. Therefore, if the humidifier 10 is placed inside a container or package holding tobacco products such as cigarettes, cigars, cigarillos or loose tobacco for pipe smoking or cigarette rolling, the tobacco can receive the vapour and be moisturised.

Water is a particularly convenient substance for the moisturising of tobacco, but other substances with suitably properties for the necessary storage and vapour release can be used. However, for the sake of convenience and conciseness, water is used as an example in the following description.

The humidifier 10 further comprises a second container 18 that in this example is adjacent to the first container 12 and shares a common wall 20 with the first container 12. The second container 18 is filled with an aqueous solution 21 of a salt hydrate such as sodium acetate, which is sealed inside the second container 18. The salt hydrate solution 21 is both supercoolable (the supercooling being made possible by the confinement of a fixed volume of the salt hydrate solution within the second container) and crystallisable.

A confined quantity of salt hydrate solution such as this can act as a heat source. From the supercooled liquid state, which exists at room temperature, the salt hydrate solution can be caused to crystallise. The crystallisation process releases heat energy via the latent heat of crystallisation. The heat is released quickly, over a period of minutes depending on the amount of salt hydrate solution. After crystallisation, the confined salt hydrate solution can be returned to its supercooled liquid state by heating it to above its melting point and allowing it to cool to room temperature. The crystallisation can then be reinitiated if desired, to release heat again. This mechanism is known for use in reusable heat packs intended to provide heat on demand without the need for any power source. The packs typically comprise a quantity of salt hydrate solution sealed inside a flexible bag, and are configured as hand warmers, warmers for food and babies' bottles, and for therapeutic heat treatments, to name just a few examples. The packs are often designed such that the crystallised salt hydrate solution can be conveniently melted by boiling the packs in water.

An activating device 22 is sealed inside the second container 18 along with the salt hydrate solution 21; this is actuable to initiate crystallisation of the supercooled salt hydrate solution 21. The device 22 may comprise a flexible metal (such as stainless steel) member, flexing of which (by manipulation through the walls of the second container) causes a single molecule of the salt hydrate solution to crystallise. This seed crystal starts a chain reaction of crystallisation that propagates through the whole volume of salt hydrate solution until it is all crystallised. Many examples of suitable activating devices are known, such as a bendable elongate member or a curved button device the curvature of which can be repeatedly tlipped between concave and convex.

US 4,077,390, US 4,587,950, \VO 02/078584 and AU 2005100469 describe assorted examples of reusable heating devices based on salt hydrate solution, with various activating devices.

In the present invention, the purpose of the sealed container 18 of salt hydrate solution 21 is to provide heat energy on demand to the moisture-releasing element 14 in the first container 12. This raises the temperature of the stored water, which increase the rate of evaporation, so that more water vapour 16 is released from the humidifier 10. Therefore, if a smoker finds that his tobacco product has become dry, and wishes to rehydrate it quickly, he can actuate the activating device 22 in the second container 18 so as to supply heat to the moisture releasing element 14 and rapidly obtain an increased rate of water vapour release.

To achieve this, the two containers 12, 18 should be arranged such that the second container is in thermal contract with the moisture-releasing element 14, so that sufficient heat can be transferred from the salt hydrate solution 21 in the second container 18 to the moisture-releasing element 14. Any partition(s) (walls of the containers) between the salt hydrate solution 21 and the moisture-releasing element 14 should be configured accordingly.

In the example of Figure 1, the first and second containers 12, 18 share a common wall 20; this is arranged to allow suitable transfer of the heat energy. The second container 18 may be made of any material that can suitably contain and seal the volume of salt hydrate solution without reacting with it, while allowing the heat energy to be transferred outwardly. The common wall 20 may be made from this material, therefore, while the first container 12 can comprise the common wall 20 made from the second container material, and a further sealer around its edge to the common wail 20 to define a reservoir volume for holding the moisture-releasing element 14. The further wall is made from a material that has the necessary permeability to water vapour.

Conveniently, both containers 12, 18 are fabricated from flexible materials. This facilitates manipulation of the activating device 22. For example, the second container 18 may be fabricated from polyethylene, polyvinyl chloride (PVC) or rubber. Other materials may also be used.

The moisture-releasing element 14 inside the first container 12 may, in a simple embodiment, take the form of a volume of liquid water, which slowly evaporates at room temperature in the known manner, or evaporates more rapidly when heated by the crystallised salt hydrate solution 21.

As an alternative to liquid water, the humidifier 10 may comprise a moisture-releasing element 14 in the form of any medium that is capable of absorbing, holding or otherwise providing a quantity or reservoir of water, and giving up the water as water vapour. For example, a paper matrix incorporating polyacrylamide granules may be used. A further example of a suitable water absorbing medium is one or more alginates. These materials are linear copolymers produced by various brown algae including seaweed (such as giant kelp) and bacteria (such as Azotobacter species), which are insoluble in water but absorb water quickly. Thus, a quantity of alginate can be soaked with water before being enclosed in the first container 12. Alginates are fully biocompatible and are used in food stuffs and medicines, so they are a particularly attractive option for use with tobacco products.

Further examples of suitable materials for making the moisture-releasing element 14 include micro-crystalline cellulose, ispaghula husk, saturated silica gel, rice, starch gels, pectins, natural sponge, synthetic sponge, clay materials such as sepeolite, bentonite or the like, gelatin, agar agar, and modified cellulosic gums. Combinations of these materials may also be used. As in the previous examples, a portion of the relevant material is soaked with water before enclosure in the first container 12.

However, the invention is not limited to these particular examples of water absorbing media for the moisture-releasing element. Any material which has the necessary properties to allow it to absorb an adequate amount of water and then release the water as water vapour by evaporation can be used.

For either liquid water or water held in a water-absorbing material, the water need not be plain water. An aqueous salt solution may be used instead, which can give control over the evaporation by determining the humidity level of the surrounding environment below which the water vapour will be released. Other non-volatile liquid ingredients such as glycerol (glycerine), or propylene glycol, may alternatively be used, as these also control evaporation of the water.

Additionally or alternatively, the moisture-releasing element may also contain one or more water-soluble additive that can be carried through the permeable first container by the water vapour and hence delivered to the tobacco. These may include flavourants and fragrances. For example, a moisture-releasing element containing menthol can be used to impart a menthol flavour and smell to the tobacco. Vanilla, coffee and other flavours may be similarly provided. In this way, the consumer has a mechanism for flavouring a tobacco product with flavor of their choice.

The reusable nature of the salt-hydrate solution, by repeated crystallisation and melting, permits repeated use of the humidifier to be suitable for repeated use. Activation of the heating effect will likely drive off all the water from the moisture-releasing element 14. The salt hydrate solution can then be supercooled for further heat supply, which requires that further water be provided within the first container 12. Also, it may be that all the water in the first container 12 is given out as vapour before it is deemed necessary to activate the crystallisation process. Therefore, in some embodiments, it is proposed that the humidifier 10 be configured such that the moisture-releasing element 14 is able to be rehydrated, that is, provided with a fresh supply of water. As mentioned above, this can be a new quantity of liquid water alone, or may be a new quantity of liquid water that is allowed to be absorbed by a water absorbing medium enclosed in the first container 12.

Depending on the type of permeable material used for the first container 12, rehydration may be possible simply by submerging the humidifier in a volume of liquid water and allowing the water to penetrate into the interior of the first container. For example, it may be soaked up by the water absorbing medium. Alternatively, a resealable opening may be provided in the first container 12 through which water can be introduced into the first container 12.

Figure 2 shows a cross-sectional view through a humidifier 10 configured in this way. The humidifier 10 comprises the same components as that shown in Figure 1, with the additional feature that the first container 12 is configured as an openable bag or pouch into which liquid water may be poured. The first container 12 therefore has an opening 24 provided with one or more sealing members 26 that allow the first container 12 to be opened and closed. In this example, the first container 12 contains no water absorbing medium, and is therefore simply refilled with liquid water. In examples including a water absorbing medium, liquid water may be poured through the opening 24, or the humidifier 10 can be placed in a volume of water while the first container 12 is open so that water can soak into the water absorbing medium.

Other ways of allowing water to be introduced into die first container 12 may be used instead. For example, the first container 12 may comprise a valve through which water can be injected into the first container 12.

Humidifiers which allow rehydration of the moisture-releasing element may be supplied for use to the consumer with or without water in the first container 12.

If it is possible to rehydrate the humidifier for additional uses, it is further convenient to be able to reactivate the salt hydrate solution 14 for additional heat supply. A simple technique for this is to place the crystallised salt hydrate solution 14 in its sealed container 18 in hot or boiling water until the salt hydrate has melted. To allow this, the first container 12 and any water absorbing medium enclosed inside it must be able to withstand the hot or boiling water for a sufficiently long time and preferably on repeated occasions. These components should therefore comprise suitable materials if such use of the humidifier is intended.

In further embodiments, the first container 12 and the second container 18 may be fabricated as two independent containers (in contrast to the shared common wall 20 in Figures 1 and 2). This may make for ease of manufacture. After fabrication, and possibly also after filling with the salt hydrate solution 21 and the moisture-releasing element 14, the two containers 12, 18 are joined together, for example by gluing, in such a way as to allow good thermal transfer between the salt hydrate solution and the moisture releasing element. They will therefore probably be joined over a relatively large surface, and possibly by use of some material having good thermal conductivity.

Figure 3 shows a cross-sectional view through a humidifier 10 according to such an embodiment. Like that of Figure 1, the humidifier 10 comprises a first container 12 filled with a moisture-releasing element 14 and a second container 18 filled with salt hydrate solution 21. However, the first and second containers 12, 18 are independent and do not share a common wall. Instead, they are adjacent to one another and coupled together over a substantial surface area using a bonding material 28 that provides adequate thermal transfer. The bonding material 28 may be a permanent bonding material to provide a humidifier 10 as a single unit. Alternatively, the bonding material 28 may be a non-permanent bonding material (for example re-useable adhesive or hook-and-loop fastening), allowing the two containers 12, 18 to be separated from one another and rejoined later. This allows the salt hydrate solution 21 in the second container 18 to be heated for melting without the first container 12 being present, so that the materials of the first container are not limited to those which can withstand the heating process. Also, the moisture-releasing properties of the first container 12 can be used without the second container 18 being present. This allows the second container 18 to be kept safe until needed, or to be removed from the tobacco product package or container after its heating ability has been utilised. Also, it may be more convenient to rehydrate the first container 12 if it is separate from the second container 18.

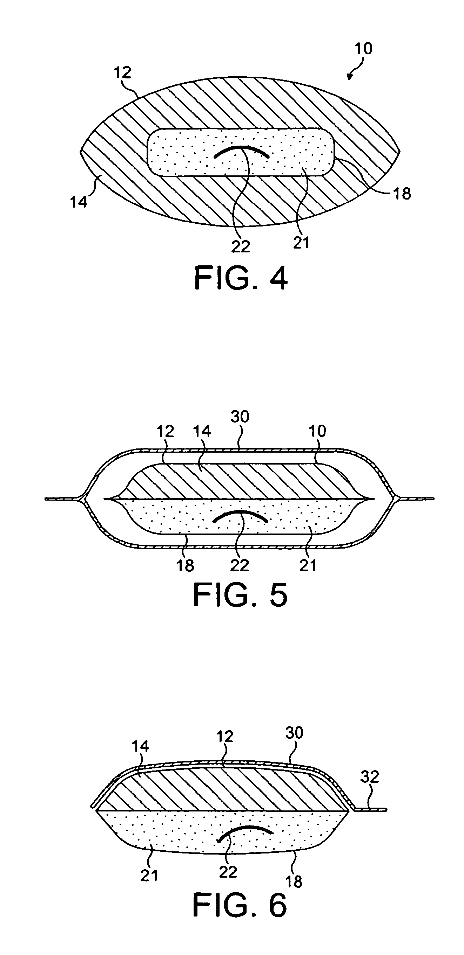

Figure 4 shows a cross-sectional view through a humidifier according to a further alternative configuration. Again, the humidifier 10 comprises a first container 12 enclosing the moisture releasing element 14, and a second container 18 containing salt-hydrate solution 21 and an activating device 22. However, in this example, the second container 18 is contained within the first container 12. This provides a maximum surface area for thermal transfer from the salt hydrate solution 21 to the moisture-releasing element 14, so gives very efficient heating and evaporation. The first container 12 and any water-absorbing medium in it should be flexible enough to allow the activating device 22 to be operated through both the first container 12 and the second container 18. A transparent first container 12 and moisture-releasing element 14 (such as liquid water) may facilitate locating of the activating device, although tough alone may be sufficient.

In this example, the first container 12 may be configured to be openable, like the embodiment of Figure 2. In addition to allowing rehydration, this also allows the second container 18 to be taken out of the first container 12 so that the salt hydrate solution 21 can be more conveniently melted for re-use.

A humidifier according to embodiments of the present invention may be supplied for use at a later time. It may be provided as an individual item, or may be supplied together with a tobacco product, for example included with the tobacco product inside the product's packaging. As mentioned above, some embodiments allow the humidifier to be supplied width no water, for hydration before use by the user. For embodiments including water, it is desirable to prevent or reduce loss of the water by evaporation before the humidifier is put to use.

Figure 5 shows a cross-sectional view through an example humidifier according to an embodiment that addresses this issue. To retain the water inside the first container and inhibit release of water vapour, the humidifier 10 is packaged inside an outer layer 30 that is substantially impermeable to the passage of water vapour. The water vapour is thereby retained substantially within the humidifier 10 for later release. The outer layer 30 in this example comprises a sealed bag, pouch, envelope or sachet that completely encloses the humidifier 10 and forms a water-tight barrier between the humidifier 10 and its surrounding environment. Any suitably impermeable material may be used for the outer layer, including thin flexible plastics materials, foils, and plasticised foils. The outer layer is to be removed from the humidifier 10 before use, and is therefore preferably configured for easy opening and removal. For example, the outer layer 30 may be made from a flexible plastics material configured as a sachet inside which the hydrated humidifier is sealed. The sachet may be of the type commonly used to provide individual portions of condiments in catering establishments, since these are simple and economical to manufacture, easy to open and remove, and use known packaging technology. The sachet is sealed across each end by sealing lines made for example by plastics welding. Portions of the sachet material extend a short distance beyond the sealing lines and terminate in serrated edges, which facilitate tearing open of the sachet for removal of the humidifier 10 prior to use. The outer surface of the sachet (or other type of outer layer) may be printed with any desired design, for example a brand name and instructions for use of the humidifier 10.

Figure 5 shows a humidifier 10 like that of Figure 1 enclosed in an outer layer 30. However, any humidifier according to the present invention can be packaged in this way if desired.

Figure 6 shows a cross-sectional view through a humidifier 10 according to a further embodiment, which is again provided with an impermeable outer layer 30 to prevent water vapour loss before use. In this example, however, the humidifier has a construction in which the first container 12 and the second container 18 are adjacent to one another, so that not all the outer surface of the humidifier 10 provides release of water vapour. In such a case, the outer layer 30 may be disposed only over that part or parts of the first container that are exposed and made from permeable material. For example, the outer layer 30 may comprise a layer of suitable impermeable material that is adhered over the permeable material and able to be peeled away from the permeable material to allow release of water vapour, as shown in Figure 6. A non-permanent adhesive may be used to adhere the outer layer 30 over the permeable material so as to seal in the water vapour, for example. A pull tab 32 extending from the edge of the outer layer 30 may be provided for grasping by the user to facilitate removal of the outer layer 30.

Although described in the context of the hydration of tobacco products such as cigarettes, cigars and loose tobacco, humidifiers according to the present invention are equally applicable to other products which may require hydration by the provision of water vapour.

Humidifiers according to the invention may comprise further features in various combinations. For example, the moisture-releasing element may comprise a gel configured for the controlled release of moisture. Humidifiers comprising such gels are described in our co-pending application GB 0710784.0 "Controlled moisture release humidifier for use with tobacco products", filed in the United Kingdom on 5th June 2007. Also, the first container may be fabricated from a thermo-mechanically expanded polymer membrane material such as polytetrafluoroethylene, polybutylene terephthalate or polyethylene oxide. Humidifiers including these materials are described in our co-pending application GB 0710773.3 "Humidifying capsule for use with tobacco products", tiled in the United Kingdom on 5th June 2007. If the humidifier is not intended for re-use, the first container may be fabricated from a semi-permeable one-way membrane that lets water vapour out but prevents water vapour or liquid from entering the moisture-releasing element. Humidifiers including membranes of this type are described in GB 0710778.2 "Disposable humidifier for use with tobacco products", filed in the United Kingdom on 5th June 2007.

## Claims

1. A humidifier (10) for use with tobacco products, comprising:
a first container (12) permeable to the passage of moisturising vapour and configured to enclose a moisture-releasing element (14) that emits moisturising vapour; **characterised by**
a second container (18) arranged for thermal energy transfer to a moisture-releasing element in the first container, the second container having sealed therein:
a supercoolable salt hydrate solution (21); and
an activating device (22) actuable to initiate crystallisation of the salt hydrate solution, thereby releasing heat and supplying it to a moisture-releasing element enclosed in the first container.

2. A humidifier according to claim 1, in which the first container is permeable to the passage of water vapour.

3. A humidifier according to claim 1 or claim 2, further comprising a moisture-releasing element enclosed in the first container, wherein the moisture-releasing element preferably emits water vapour.

4. A humidifier according to claim 4, in which the moisture-releasing element comprises:
(a) a reservoir of liquid water; or,
(b) water absorbed in a water-absorbing medium, which is preferably one or more of polyacrylamide granules, an alginate, microcrystalline cellulose, ispaghula husk, saturated silica gel, rice, a starch gel, a pectin, natural sponge, synthetic sponge, a sepeolite, bentonite or other clay material, gelatin, agar agar, and a modified cellulosic gum.

5. A humidifier according to any preceding claim, in which the first and/ or second container is flexible.

6. A humidifier according to any one of claims 1 to 5, in which the first container and the second container are adjacent to one another.

7. A humidifier according to claim 6, in which the first container and the second container are separate and coupled together by a bonding material, which is preferably reversibly releasable to allow the first container and the second container to be coupled and uncoupled.

8. A humidifier according to claim 6, in which the first container and the second container share a common wall.

9. A humidifier according to any one of claims 1 to 5, in which the second container is inside the first container.

10. A humidifier according to any preceding claim, in which the humidifier is configured for more than one release of heat from the salt hydrate solution.

11. A humidifier according to claim 10, in which the first container and any moisture-releasing element enclosed in the first container are able to withstand immersion in hot water for the purpose of melting the salt hydrate solution after crystallisation.

12. A humidifier according to any preceding claim, in which the moisture-releasing element can be rehydrated.

13. A humidifier according to claim 12, in which the first container can be opened and closed for the purpose of introducing liquid into the first container.

14. A humidifier according to any preceding claim, further comprising a removable outer layer (30) that is substantially impermeable to the passage of water and water vapour, the outer layer disposed over at least the first container and intended to be removed before use of the humidifier, wherein the removable outer layer preferably comprises:
(a) an outer wrapper enclosing both the first container and the second container; or,
(b) a peelable layer adhered over at least part of the outer surface of the first container.

15. A package for tobacco products containing one or more tobacco products and a humidifier according to any one of claims 1 to 14.

## Patentansprüche

1. Befeuchter (10) zur Verwendung mit Tabakprodukten, der Folgendes umfasst:
einen ersten Behälter (12), der für den Durchtritt von Befeuchtungsdampf durchlässig ist und dazu konfiguriert ist, ein Feuchtigkeit freisetzendes Element (14), das Befeuchtungsdampf abgibt, einzukapseln; **gekennzeichnet durch**
einen zweiten Behälter (18), der zur Wärmeenergieübertragung an ein Feuchtigkeit freisetzendes Element in dem ersten Behälter eingerichtet ist, wobei der zweite Behälter darin versiegelt Folgendes aufweist:
eine unterkühlbare Salzhydratlösung (21) und
eine Aktivierungsvorrichtung (22), die dazu betätigbar ist,
eine Kristallisation der Salzhydratlösung einzuleiten,
wodurch Wärme freigesetzt wird und diese einem Feuchtigkeit freisetzenden Element, das in dem ersten Behälter eingekapselt ist, zugeführt wird.

2. Befeuchter nach Anspruch 1, wobei der erste Behälter für den Durchtritt von Wasserdampf durchlässig ist.

3. Befeuchter nach Anspruch 1 oder 2, der weiterhin ein Feuchtigkeit freisetzendes Element umfasst, das in dem ersten Behälter eingekapselt ist, wobei das Feuchtigkeit freisetzende Element vorzugsweise Wasserdampf abgibt.

4. Befeuchter nach Anspruch 4, wobei das Feuchtigkeit freisetzende Element Folgendes umfasst:
(a) ein Reservoir von flüssigem Wasser und
(b) Wasser, das in einem wasserabsorbierenden Medium absorbiert ist, bei dem es sich vorzugsweise um eines oder mehrere von Polyacrylamidgranulat, einem Alginat, mikrokristalliner Cellulose, indischen Flohsamenschalen, gesättigtem Silicagel, Reis, einem Stärkegel, einem Pektin, Naturschwamm, Kunstschwamm, einem Sepiolith, Bentonit oder einem anderen Tonmaterial, Gelatine, Agar-Agar und/oder einem modifizierten Cellulosegummi handelt.

5. Befeuchter nach einem vorhergehenden Anspruch, wobei der erste und/oder der zweite Behälter flexibel ist bzw. sind.

6. Befeuchter nach einem der Ansprüche 1 bis 5, wobei der erste Behälter und der zweite Behälter aneinander angrenzen.

7. Befeuchter nach Anspruch 6, wobei der erste Behälter und der zweite Behälter getrennt sind und durch ein Bindungsmaterial miteinander verbunden sind, das vorzugsweise reversibel lösbar ist, um zu ermöglichen, den ersten Behälter und den zweiten Behälter zu verbinden und zu trennen.

8. Befeuchter nach Anspruch 6, wobei der erste Behälter und der zweite Behälter eine gemeinsame Wand teilen.

9. Befeuchter nach einem der Ansprüche 1 bis 5, wobei der zweite Behälter im Inneren des ersten Behälters ist.

10. Befeuchter nach einem vorhergehenden Anspruch, wobei der Befeuchter für mehr als eine Freisetzung von Wärme aus der Salzhydratlösung konfiguriert ist.

11. Befeuchter nach Anspruch 10, wobei der erste Behälter und ein etwaiges Feuchtigkeit freisetzendes Element, das in dem ersten Behälter eingekapselt ist, dazu fähig sind, einem Eintauchen in heißes Wasser zum Zwecke des Schmelzens der Salzhydratlösung nach der Kristallisation standzuhalten.

12. Befeuchter nach einem vorhergehenden Anspruch, wobei das Feuchtigkeit freisetzende Element rehydriert werden kann.

13. Befeuchter nach Anspruch 12, wobei der erste Behälter zum Zwecke des Einbringens von Flüssigkeit in den ersten Behälter geöffnet und geschlossen werden kann.

14. Befeuchter nach einem vorhergehenden Anspruch, der weiterhin eine entfernbare Außenschicht (30) umfasst, die für den Durchtritt von Wasser und Wasserdampf im Wesentlichen durchlässig ist, wobei die Außenschicht über mindestens dem ersten Behälter angeordnet ist und dazu vorgesehen ist, vor Gebrauch des Befeuchters entfernt zu werden, wobei die entfernbare Außenschicht vorzugsweise Folgendes umfasst:
(a) eine äußere Hülle, die sowohl den ersten Behälter als auch den zweiten Behälter einkapselt; oder
(b) eine abziehbare Schicht, die über mindestens einen Teil der Außenfläche des ersten Behälters geklebt ist.

15. Verpackung für Tabakprodukte, die ein oder mehrere Tabakprodukte und einen Befeuchter nach einem der Ansprüche 1 bis 14 enthält.

## Revendications

1. Humidificateur (10) devant être utilisé avec des produits du tabac, comprenant :
un premier récipient (12) perméable aux vapeurs hydratantes et configuré pour renfermer un élément libérant l'humidité (14) qui émet une vapeur hydratante ; **caractérisé par**
un deuxième récipient (18) agencé pour le transfert d'énergie thermique à un élément libérant l'humidité dans le premier récipient, les éléments suivants étant scellés dans le deuxième récipient :
une solution de sel hydraté pouvant être surfondue (21) ; et
un dispositif d'activation (22) pouvant être actionné pour initier la cristallisation de la solution de sel hydraté, en libérant ainsi de la chaleur et en la fournissant à un élément libérant l'humidité enfermé dans le premier récipient.

2. Humidificateur selon la revendication 1, dans lequel le premier récipient est perméable à la vapeur d'eau.

3. Humidificateur selon la revendication 1 ou la revendication 2, comprenant en outre un élément libérant l'humidité enfermé dans le premier récipient, dans lequel l'élément libérant l'humidité émet de préférence de la vapeur d'eau.

4. Humidificateur selon la revendication 4, dans lequel l'élément libérant l'humidité comprend :
(a) un réservoir d'eau liquide ; ou
(b) de l'eau absorbée dans un milieu absorbant l'eau, qui est de préférence un ou plusieurs des éléments suivants : granules de polyacrylamide, un alginate, cellulose microcristalline, enveloppe d'ispaghula, gel de silice saturé, riz, un gel d'amidon, une pectine, éponge naturelle, éponge synthétique, une sépéolite, bentonite ou autre argile, gélatine, agar-agar, et une gomme cellulosique modifiée.

5. Humidificateur selon l'une quelconque des revendications précédentes, dans lequel le premier et/ou le deuxième récipient sont flexibles.

6. Humidificateur selon l'une quelconque des revendications 1 à 5, dans lequel le premier récipient et le deuxième récipient sont contigus.

7. Humidificateur selon la revendication 6, dans lequel le premier récipient et le deuxième récipient sont séparés et couplés ensemble par un matériau adhésif qui est de préférence décollable de façon réversible pour permettre le couplage et le découplage du premier récipient et du deuxième récipient.

8. Humidificateur selon la revendication 6, dans lequel le premier récipient et le deuxième récipient partagent une paroi commune.

9. Humidificateur selon l'une quelconque des revendications 1 à 5, dans lequel le deuxième récipient est à l'intérieur du premier récipient.

10. Humidificateur selon l'une quelconque des revendications précédentes, dans lequel l'humidificateur est configuré pour plus d'une libération de chaleur de la solution de sel hydraté.

11. Humidificateur selon la revendication 10, dans lequel le premier récipient et tout élément libérant de l'humidité enfermé dans le premier récipient sont capables de supporter l'immersion dans de l'eau chaude aux fins de faire fondre la solution de sel hydraté après cristallisation.

12. Humidificateur selon l'une quelconque des revendications précédentes, dans lequel l'élément libérant l'humidité peut être réhydraté.

13. Humidificateur selon la revendication 12, dans lequel le premier récipient peut être ouvert et fermé afin d'introduire un liquide dans le premier récipient.

14. Humidificateur selon l'une quelconque des revendications précédentes, comprenant en outre une couche extérieure amovible (30) qui est essentiellement imperméable à l'eau et à la vapeur d'eau, la couche extérieure étant disposée par-dessus au moins le premier récipient et prévue pour être enlevée avant l'utilisation de l'humidificateur, dans lequel la couche extérieure amovible comprend de préférence :
(a) une enveloppe extérieure enfermant à la fois le premier récipient et le deuxième récipient ; ou
(b) une couche décollable collée sur au moins une partie de la surface extérieure du premier récipient.

15. Emballage pour produits du tabac contenant un ou plusieurs produits du tabac et un humidificateur selon l'une quelconque des revendications 1 à 14.
